# EUROPEAN PATENT APPLICATION

(11) **EP 2 543 350 A1**
(43) Date of publication of application: **09.01.2013**
(21) Application number: 10849901.3
(22) Date of filing: 17.09.2010
(51) Int. Cl.: A61F 13/02, A61L 15/22, C08F 20/06, B23B 27/00, A61K 31/78

(54) **ADHESIVE HYDROUS GEL SHEET**

(30) Priority: 10.08.2010 KR 20100076789; 14.04.2010 KR 20100034185
(71) Applicant: Kim, Bongjo, Guri Si, Gyeonggi-Do 471-070 (KR)
(72) Inventor: Kim, Bongjo, Guri Si, Gyeonggi-Do 471-070 (KR)
(74) Representative: Cabinet Plasseraud
(86) International application number: PCT/KR2010/006437
(87) International publication number: WO 2011/129497

(57) **Abstract**

The present invention relates to a hydrous gel sheet including at least two laminated layers of a body-side surface layer and an environment-side surface layer, each of which is made of a hydrogel obtained from the reaction of polyacrylic acid or a salt thereof and multivalent metal ions, wherein the polyacrylic acid or the salt thereof used for forming a gel matrix of the environment-side surface layer is greater in weight-average molecular weight than the polyacrylic acid or the salt thereof used for forming a gel matrix of the body-side surface layer. Since a support body such as a nonwoven fabric is not used for the environment-side surface, but rather a support body made of hydrous gel is formed on the environment-side surface, the adhesive hydrous gel sheet according to the present invention, which is used for adhering to the human body, has excellent wear and handling properties as well as excellent stretching and deformation-absorbing properties, and also exhibits an improved appearance, aging stability, and durability.

## Description

### [Technical Field]

The present invention relates to an adhesive hydrous gel sheet used for adhering to a human body, as a cosmetic product, a medical tool, a cooling tool, a cataplasm and a percutaneous absorption type patch.

### [Background Art]

Generally, factors required for a hydrous gel sheet include wear properties or feelings (stretching property, deformation-absorbing property, adhesiveness, and exfoliation), handling properties (support property, handling easiness, and aging stability), effective ingredient release property, and appearance. However, a support body such as a nonwoven fabric is used at the environment-side surface in the conventional hydrous gel sheets; so they have a problem of lacks in stretching and deformation-absorbing properties. Therefore, trials without using the support body have variously tried. For example, there were a case of forming a support base with gelatin (Patent Document 1), and a case of performing a non-adhesive treatment at the environment-side surface (Patent Document 2). There were also cases of suggesting simple two-layered hydrous gel sheets (Patent Documents 3 and 4).
Patent Document 1: Japanese Patent Laid Open No. Hei 3-167117 and Hei 3-86808
Patent Document 2: Japanese Patent Laid Open No. 2000-297009 Patent Document 3: Japanese Patent Laid Open No. 2003-252753
Patent Document 4: Japanese Patent No. 4327958 (Japanese Patent Laid Open No. 2001-240535)

### [Disclosure]

### [Technical Problem]

However, in case of Patent Document 1, gelatin has problems in microorganism and the heat resistance property. The non-adhesive treatment in Patent Document 2 has also problems in the appearance of the treatment area, the stability of the non-adhesive treatment with time passing, and durability; so it is difficult to say that the treatment is available. Patent Document 3 relates to a cooling sheet in which the moisture content of each layer is different from each other. However the moisture evaporates and moves with time passing in preservation; so it is also difficult to say that it is available. Patent Document 4 also relates to a cooling sheet in which the control for the moisture evaporation rate is specialized. In the point that both of the two surfaces in the cooling sheet have adhesiveness without difference and in the point of handling properties, it is also difficult to say that the cooling sheet is available. The suggestions as mentioned above are effective for stretching and deformation-absorbing properties, but they bring out new problems; so they do not arrive at an available level.

Accordingly, the purpose of the present invention is to provide an adhesive hydrous gel sheet used for adhering to a human body, which has not a problem in appearance and has an available level in wear and handling properties, in addition to an enhancement of stretching and deformation-absorbing properties, by means of not using the support body at the surface of the hydrous gel sheet.

### [Technical Solution]

In order to achieve the purpose as mentioned above, an adhesive hydrous gel sheet according to the present invention includes at least two laminated layers of a body-side surface layer and an environment-side surface layer. In such a hydrous gel sheet, each layer is formed of a hydrogel obtained from the reaction of polyacrylic acid or a salt thereof with a multivalent metal ion, and the weight-average molecular weight of the polyacrylic acid or the salt thereof used for forming a gel matrix of the environment-side surface layer is greater than the weight-average molecular weight of the polyacrylic acid or the salt thereof used for forming a gel matrix of the human body-side surface layer.

The hydrous gel sheet according to the present invention preferably has the property that when the adhesiveness of each of the two surface layers is measured with a slope type ball tack device (30 degree of slope angle) according to the test method for an adhesive tape or adhesive sheet described in JIS Z 0237, the difference of the ball number is 1 or more.

It is preferable in the present invention that the weight-average molecular weight of the polyacrylic acid or the salt thereof used for forming the gel matrix of the environment-side surface layer is at least 500,000 greater than the weight-average molecular weight of the polyacrylic acid or the salt thereof used for forming the gel matrix of the body-side surface layer. It is also preferable that the weight-average molecular weight of the polyacrylic acid or the salt thereof used for forming one or boh of the two gel matrix is greater than 1,000,000.

It is preferable in the present invention that at least one water permeable core material or/and water impermeable core material is arranged in a layer of the two surface layers or/and between the two surface layers.

It is also preferable in the present invention that at least one of the hydrogel layers in the hydrous gel sheet includes oil gel or polysaccharide gel dispersed into the hydrogel obtained by the reaction of the polyacrylic acid or the salt thereof with the multivalent metal ion.

### [Advantageous Effects]

The adhesive hydrous gel sheet used for adhering to the human body according to the present invention is good in appearance, aging stability and durability as well as stretching, deformation-absorbing, wear, and handling properties, because it has not the support body such as a non-woven fabric but rather the support layer made of the hydrogel at the environment-side surface.

### [Description of Drawings]

The proportional rates for dimensions and figure in the drawings are different from the real objects.
Fig. 1 is a cross-sectional drawing of the adhesive hydrous gel sheet according to Example 6.
Fig. 2 is a cross-sectional drawing of the adhesive hydrous gel sheet according to Example 1.

### [Best Mode]

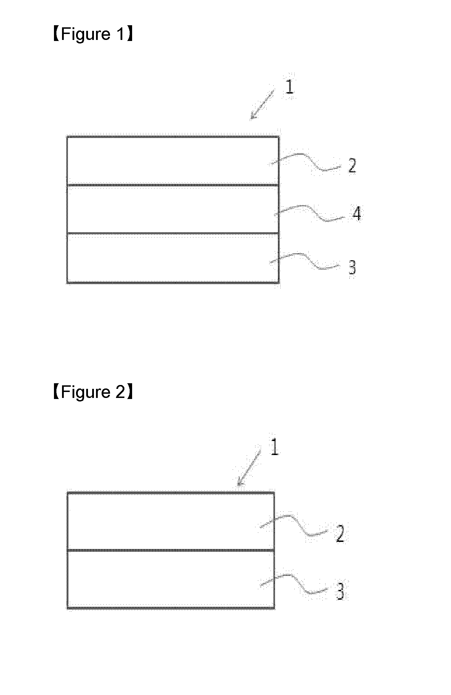

As shown in Figs. 1 and 2, an adhesive hydrous gel sheet 1 according to the present invention is formed by laminating at least two layers, namely one human body-side surface layer which is used as an adhesive layer 3, and one environment-side surface layer which is used as a support layer 2. The adhesive layer has a surface for adhering to a skin of a person and the support layer has a surface exposed to the environment. Such adhesive and support layers are formed with hydrous gel obtained by the reaction of polyacrylic acid or a salt theoreof with a multivalent metal ion. At this time, the multivalent metal ion reacts with the polyacrylic acid or the salt thereof to play a role of cross-linking agent or curing agent.

The multivalent metal ion as used in the present invention is not limited if it can react with the polyacrylic acid or the salt thereof to play a role of cross-linking agent, but it is preferable that the multivalent metal ion may be selected from the group consisting of aluminum compounds, calcium compounds and magnesium compounds. In particular, it is more preferable that the multivalent metal ion may be selected from the group consisting of compounds including three-valent aluminum ion such as aluminum hydroxide and salts thereof, synthetic aluminum silicates, metasilicate magnesium aluminates, magnesium-aluminum oxides, aluminum oxides, and dihydroxyaluminumaminoacetate.

The support and adhesive layers in the adhesive hydrous gel sheet of the present invention may also include an organic solvent miscible with water. Such an organic solvent may provide a help in dissolving and stabilizing several additives including functional materials such as cosmetics and medicines. Such an organic solvent may include monoalcohols such as ethyl alcohol and polyalcohols such as glycols and glycerins.

The base material other than the polyacrylic acid or the salt thereof as used for the gel sheet of the present invention is not limited to, but may include hydrophilic polymers such as gelatin, casein, pullulan, dextran, alginate, starch, carboxymethylcellulose (CMC) or salts thereof, methylcellulose (MC), ethylcellulose, hydroxyethylcellulose, PVA (polyvinylacetate), polyethyleneoxide, polyacrylamide, PVP (polyvinylpyrrolidone), and carboxyvinyl polymers, and polysaccharides such as agar, xanthan gum, and locust bean gum. As the needs of the case may be mixed the materials including polyalcohols, alcohols, acids, esters of fatty acids, oils, surfactants, oil gelation agents such as hydrogelation agents, dextrin palmitate, dextrin palmitate/octanoate, and dextrin myristate, adhesive agents, humectants, preservatives, fillers, antioxidants, chelating agents, perfumes, and colorants. In addition, effective and necessary ingredients for several uses may properly be mixed.

As the needs of the case, non-woven fabrics, woven fabrics and papers may be used to be buried in or between the hydrous gel layers as a water permeable core material and films of polyethylene and polypropylene and materials formed by laminating the films with the water permeable core material may be also used to be buried in or between the hydrous gel layers as a water impermeable core material. The water impermeable core material may be also used as a water permeable core material after forming pores or slits through it. Films of polyethylene and polypropylene and papers (including a release paper) may be also used as a liner.

The inventor has accomplished the basic skeleton of the present invention by obtaining the knowledge that the several problems about wear properties or feelings (stretching, deformation-absorbing properties, adhesiveness, and exfoliation), handling properties (support property, handling easiness, and aging stability), effective ingredient release property, and appearance will be solved by forming a laminating structure of at least two layers consisting of an environment-side surface layer, namely a support layer and a human body-side surface layer, namely an adhesive layer without means of surface treatment, and using as a main ingredient, higher molecular weight of polyacrylic acid or salts thereof for the support layer and lower molecular weight of polyacrylic acid or salts thereof for the adhesive layer. The difference between the weight-average molecular weights of the polyacrylic acid or the salt thereof used in the two layers is preferably 500,000 or more, more preferably 1,000,000 or more, and most preferably 3,000,000 or more. The weight-average molecular weight of the polyacrylic acid or the salt thereof used for forming at least one of the gel matrixes of the two layers is preferably 1,000,000 or more.

Therefore, the hydrous gel sheet according to the present invention has preferably the property that when the adhesiveness of each of the two surface layers is measured with a slope type ball tack device (30 degree of slope angle) according to the test method for an adhesive tape or adhesive sheet described in JIS Z 0237, the difference of the ball number is 1 or more.

The technical solution as described above may be more effective by considerations of additional amount of the polyacrylic acid or the salt thereof in each of the two layers, controlling additional amount of the cross-linking agent in each layer, adding other adhesive agents such as polyacrylate in the adhesive layer, whether each layer has a filler or not, and controlling additional amount of the filler.

However, the control of the cross-linking agent is particularly only a limited auxiliary mean, because the change of gel strength due to the movement of the metal ion with time passing, and the change of adhesiveness accompanying with it will occur.

The hydrous gel sheet of the present invention has also a water permeable core material or/and a water impermeable core material in or/and between the two layers. The selection and use of the core material may properly control the stretching property of the hydrous gel sheet and enhance the handling properties without deteriorating the deformation-absorbing property.

The hydrous gel sheet of the present invention is a gel sheet in which oil gel or polysaccharide gel is dispersed into the hydrogel in at least one layer of the two hydrogel layers, obtained by the reaction of polyacrylic acid or the salt thereof with the multivalent metal ion. Release property of an effective ingredient becomes better than the prior products by using as a main ingredient, lower molecular weight of polyarylic acid or the salt thereof in the body-side surface layer as described above, and aging stability is also enhanced by additionally forming the hybrid gel structure.

In the body-side and environment-side surface layers in the hydrous gel sheet of the present invention, the polyacrylic acid or the salt thereof is used with an amount of 1∼30 weight %, and preferably 3∼10 weight %, and the multivalent metal ion compound is used with an amount of 0.01∼5 weight %, and preferably 0.1∼2 weight %. Water may be included in it with an amount of 1∼95 weight %, and preferably 30∼90 weight %. In case of each layer including a mixture of the hydrogel and an oil gel, the ratio of the hydrogel and the oil gel is not limited to, but preferably 70:30∼99:1. In case of each layer including a mixture of the hydrogel and a polysaccharide, the ratio of the hydrogel and the polysaccharide is not limited to, but preferably 55:45∼99:1.

### [Mode for Invention]

Hereinafter, the present invention will be exemplified with Examples, but its scope should not be limited to the Examples.

### Example

Composition of each example according to the present invention will be shown in Table 1, below.

**Table 1a**

| | Example 1 | | Example 2 | |
|---|---|---|---|---|
| | Body-side Layer | Environment-side Layer | Body-side Layer | Environment-side Layer |
| Sodium Polyacrylate (Mw: 4,000,000∼5,000,000) | | 8.00 | | |
| Sodium Polyacrylate (Mw: 2,000,000) | | | | |
| Sodium Polyacrylate (Mw: 1,500,000) | | | | |
| Sodium Polyacrylate (Mw: 1,000,000) | | | | 8.0 |
| Sodium Polyacrylate (Mw: 650,000) | | | | |
| Sodium Polyacrylate (Mw: 150,000) | 9.0 | | 9.0 | |
| Glycerin | 10.0 | 10.0 | 10.0 | 10.0 |
| Metasilicate Magnesium Aluminate | 0.35 | 0.35 | 0.35 | 0.35 |
| Tartaric acid | 0.2 | 0.2 | 0.2 | 0.2 |
| Olive oil | 0.5 | 0.5 | 0.5 | 0.5 |
| Sodium EDTA | 0.3 | 0.3 | 0.3 | 0.3 |
| Water | 79.65 | 80.65 | 79.65 | 80.65 |
| Oil gel | | | | |
| Agar gel | | | | |

In the above table, the molecular weights of the polymers are weight-average molecular weight (Mw), and the amount of each ingredient is weight %. Hereinafter, the same notation will be applied.

**Table 1b**

| | Example 3 | | Example 4 | |
|---|---|---|---|---|
| | Body-side Layer | Environment-side Layer | Body-side Layer | Environment-side Layer |
| Sodium Polyacrylate (Mw: 4,000,000∼5,000,000) | | | | |
| Sodium Polyacrylate (Mw: 2,000,000) | | | | |
| Sodium Polyacrylate (Mw: 1,500,000) | | | | 8.0 |
| Sodium Polyacrylate (Mw: 1,000,000) | | | | |
| Sodium Polyacrylate (Mw: 650,000) | | 9.0 | 9.0 | |
| Sodium Polyacrylate (Mw: 150,000) | 9.0 | | | |
| Glycerin | 10.0 | 10.0 | 10.0 | 10.0 |
| Metasilicate Magnesium Aluminate | 0.35 | 0.35 | 0.35 | 0.35 |
| Tartaric acid | 0.2 | 0.2 | 0.2 | 0.2 |
| Olive oil | 0.5 | 0.5 | 0.5 | 0.5 |
| Sodium EDTA | 0.3 | 0.3 | 0.3 | 0.3 |
| Water | 79.65 | 79.65 | 79.65 | 80.65 |
| Oil gel | | | | |
| Agar gel | | | | |

**Table 1c**

| | Example 5 | | Example 8 | |
|---|---|---|---|---|
| | Body-side Layer | Environment-side Layer | Body-side Layer | Environment-side Layer |
| Sodium Polyacrylate (Mw: 4,000,000∼5,000,000) | | | | |
| Sodium Polyacrylate (Mw: 2,000,000) | | 8.0 | | 8.0 |
| Sodium Polyacrylate (Mw: 1,500,000) | | | | |
| Sodium Polyacrylate (Mw: 1,000,000) | 8.0 | | | |
| Sodium Polyacrylate (Mw: 650,000) | | | 9.0 | |
| Sodium Polyacrylate (Mw: 150,000) | | | | |
| Glycerin | 10.0 | 10.0 | 10.0 | 10.0 |
| Metasilicate Magnesium Aluminate | 0.35 | 0.35 | 0.35 | 0.35 |
| Tartaric acid | 0.2 | 0.2 | 0.2 | 0.2 |
| Olive oil | 0.5 | 0.5 | 0.5 | 0.5 |
| Sodium EDTA | 0.3 | 0.3 | 0.3 | 0.3 |
| Water | 80.65 | 80.65 | 79.65 | 80.65 |
| Oil gel | | | o | |
| Agar gel | | | | |

**Table 1d**

| | Example 9 | | Comparative Example 1 | |
|---|---|---|---|---|
| | Body-side Layer | Environment-side Layer | Body-side Layer | Environment-side Layer |
| Sodium Polyacrylate (Mw: 4,000,000∼5,000,000) | | | | |
| Sodium Polyacrylate (Mw: 2,000,000) | | 8.0 | | |
| Sodium Polyacrylate (Mw: 1,500,000) | | | | |
| Sodium Polyacrylate (Mw: 1,000,000) | | | | |
| Sodium Polyacrylate (Mw: 650,000) | 9.0 | | 9.0 | 9.0 |
| Sodium Polyacrylate (Mw: 150,000) | | | | |
| Glycerin | 10.0 | 10.0 | 10.0 | 10.0 |
| Metasilicate Magnesium Aluminate | 0.35 | 0.35 | 0.35 | 0.35 |
| Tartaric acid | 0.2 | 0.2 | 0.2 | 0.2 |
| Olive oil | 0.5 | 0.5 | 0.5 | 0.5 |
| Sodium EDTA | 0.3 | 0.3 | 0.3 | 0.3 |
| Water | 79.65 | 80.65 | 79.65 | 79.65 |
| Oil gel | | | | |
| Agar gel | o | | | |

**Table 1e**

| | Comparative Example 2 | | Comparative Example 3 | |
|---|---|---|---|---|
| | Body-side Layer | Environment-side Layer | Body-side Layer | Environment-side Layer |
| Sodium Polyacrylate (Mw: 4,000,000∼5,000,000) | | | | |
| Sodium Polyacrylate (Mw: 2,000,000) | | | 8.0 | 8.0 |
| Sodium Polyacrylate (Mw: 1,500,000) | | | | |
| Sodium Polyacrylate (Mw: 1,000,000) | 8.0 | 8.0 | | |
| Sodium Polyacrylate (Mw: 650,000) | | | | |
| Sodium Polyacrylate (Mw: 150,000) | | | | |
| Glycerin | 10.0 | 10.0 | 10.0 | 10.0 |
| Metasilicate Magnesium Aluminate | 0.35 | 0.35 | 0.35 | 0.35 |
| Tartaric acid | 0.2 | 0.2 | 0.2 | 0.2 |
| Olive oil | 0.5 | 0.5 | 0.5 | 0.5 |
| Sodium EDTA | 0.3 | 0.3 | 0.3 | 0.3 |
| Water | 80.65 | 80.65 | 80.65 | 80.65 |
| Oil gel | | | | |
| Agar gel | | | | |

**Table 1f**

| | Comparative Example 4 | |
|---|---|---|
| | Human body side layer | Environmental side layer |
| Sodium Polyacrylate (Mw: 4,000,000∼5,000,000) | 8.0 | 8.0 |
| Sodium Polyacrylate (Mw: 2,000,000) | | |
| Sodium Polyacrylate (Mw: 1,500,000) | | |
| Sodium Polyacrylate (Mw: 1,000,000) | | |
| Sodium Polyacrylate (Mw: 650,000) | | |
| Sodium Polyacrylate (Mw: 150,000) | | |
| Glycerin | 10.0 | 10.0 |
| Metasilicate Magnesium Aluminate | 0.35 | 0.35 |
| Tartaric acid | 0.2 | 0.2 |
| Olive oil | 0.5 | 0.5 |
| Sodium EDTA | 0.3 | 0.3 |
| Water | 80.65 | 80.65 |
| Oil gel | | |
| Agar gel | | |

### Examples 1∼5 and Comparative Examples 1∼4

Each ingredient was selected to make each composition for the human body-side layer and the environment-side layer according to Table 1, and the ingredients were mixed in accordance with a conventional method. The compositions were respectively coated on a polyethylene liner with the rate of 500 g/m². Immediately, both sides of the two gel sheets were pressed with an appropriate pressure to adhere to each other. It was then placed at room temperature for 3 days, and was cut to wrinkle pack sheet type.

### Example 6

Each ingredient was selected to make each composition for the human body-side layer and the environment-side layer according to the composition of Example 4 in Table 1, and the ingredients were mixed in accordance with a conventional method. The compositions were respectively coated on a polyethylene liner with the rate of 500 g/m². Immediately, a core material was inserted between both sides of the two gel sheets which were then pressed with an appropriate pressure to adhere to each other. It was then placed at room temperature for 3 days, and was cut to wrinkle pack sheet type. The core material was a hydrophilic non-woven fabric (natural cotton) with porous property and the density of 18 g/m².

### Example 7

Each ingredient was selected to make each composition for the human body-side layer and the environment-side layer according to the composition of Example 5 in Table 1, and the ingredients were mixed in accordance with a conventional method. The compositions were respectively coated on a polyethylene liner with the rate of 500 g/m². Immediately, a core material was inserted between both sides of the two gel sheets which were then pressed with an appropriate pressure to adhere to each other. It was then placed at room temperature for 3 days, and was cut to wrinkle pack sheet type. The core material was formed by laminating polyethylene sheet with a hydrophilic non-woven fabric (natural cotton) with the density of 40 g/m².

### Example 8

Each ingredient was selected to make each composition for the human body-side layer and the environment-side layer according to Table 1, and the ingredients were mixed in accordance with a conventional method. The composition for the environment-side layer was coated on a polyethylene liner with the rate of 500 g/m². The composition for the body-side layer including a mixture of the hydrogel and an oil gel with the ratio of 90:10 was then coated on a polyethylene liner with the rate of 500 g/m². Immediately, both sides of the two gel sheets were pressed with an appropriate pressure to adhere to each other. It was then placed at room temperature for 3 days, and was cut to wrinkle pack sheet type. The method for preparing the oil gel was to add 10g of dextrin palmitate into 90g of glyceryl tri(2-ethylhexanoate), to disperse dextrin palmitate at room temperature and to slowly heat and dissolve dextrin palmitate completely. It was then displaced at room temperature for 1 or 2 days for gelation.

### Example 9

Each ingredient was selected to make each composition for the human body-side layer and the environment-side layer according to Table 1, and the ingredients were mixed in accordance with a conventional method. The composition for the environment-side layer was coated on a polyethylene liner with the rate of 500 g/m². The composition for the body-side layer including a mixture of the hydrogel and agar gel with the ratio of 90:10 was then coated on a polyethylene liner with the rate of 500 g/m². Immediately, both sides of the two gel sheets were pressed with an appropriate pressure to adhere to each other. It was then placed at room temperature for 3 days, and was cut to wrinkle pack sheet type. The method for preparing the agar gel was to add 2g of agar into 98g of water and to slowly heat and dissolve agar completely. It was then displaced at room temperature or at a cooling place for 1 or 2 days for gelation.

For the body-side and environment-side surfaces in the hydrous gel sheet obtained by Examples and Comparative Examples as described above, adhesiveness tests in use of a slop type ball tack device (30 degree of slope angle) according to the description in JIS Z 0237 'test method for an adhesive tape or adhesive sheet' were performed and the obtained results were shown in Table 2, below.

**Table 2**

| | Body-side Surface | Environment-side Surface |
|---|---|---|
| Example 1 | 12 | 2 |
| Example 2 | 10 | 5 |
| Example 3 | 12 | 6 |
| Example 4 | 9 | 5 |
| Example 5 | 7 | 3 |
| Example 6 | 8 | 4 |
| Example 7 | 7 | 3 |
| Example 8 | 9 | 4 |
| Example 9 | 7 | 3 |
| Comparative Example 1 | 8 | 8 |
| Comparative Example 2 | 7 | 7 |
| Comparative Example 3 | 3 | 3 |
| Comparative Example 4 | 2 | 2 |

| | | |
|---|---|---|
| The number means ball number. | | |

The Examples may show that the human body-side surface has an appropriate adhesiveness as required and the environment-side surface has a certain adhesiveness which does not bother the hydrous gel sheet to be used appropriately, and therefore the hydrous gel sheet has no problem in actual use. However, the Comparative Examples showed that their hydrous gel sheets were bad in the handling properties and wear properties because there were no difference in the adhesiveness of the two surfaces of the human body side and the environmental side, or because the adhesiveness of the human body side surface was weak.

Meanwhile, use tests that participants used the hydrous gel sheets according to the Example of the present invention and the Comparative Example were performed.

### 1. Test Participants

Twenty of females with an age range of 20 to 50 years and normal skin, who do not have functional disorders, symptoms to be diagnosed to skin diseases, defects and deformations in face.

### 2. Test Objects

### Example 1 and Comparative Example 1

### 3. Test Method

The participants washed their faces and applied the wrinkle pack sheet of the test objects to their faces for 15 minutes before going to bed. The next day morning, the effects were evaluated. The participants were divided into two groups, each of which consisted of 10 persons. At the first day, the participants in the first group put the hydrous gel sheet of Example 1 on the left side of their face and put the hydrous gel sheet of Comparative Example 1 on the right side of their face, and the participants in the second group put that of Example 1 on the right side of their face and put that of Comparative Example 1 on the left side of their face. Then at the second day, the hydrous gel sheets were applied at the opposite side to the side applied at the first day.

### 4. Evaluation

The numbers in parentheses are evaluation scores. Evaluation was performed with total scores for 20 persons for two days.
A. About handling properties
   ·Very good (3) ·Good (2) ·A little uncomfortable (1) ·Very uncomfortable (0)
B. About wear properties (contact feeling, adhesiveness, exfoliation)
   ·Very good (3) ·Good (2) ·A little uncomfortable (1) ·Very uncomfortable (0)
C. Moisturizing Effect
   ·Very good (3) ·Good (2) ·A little uncomfortable (1) ·Very uncomfortable (0)

### 5. Results

### Example 1 A (115) B(110) C(106)

### Comparative Example 1 A (25) B (42) C (88)

### 6. Observations

The Example was evaluated to be excellent in handling properties (support property, handling easiness) and wear properties (contact feeling, adhesiveness, exfoliation). Meanwhile, both of the Example and the Comparative Example were evaluated to be good in the moisturizing effect. These evaluations are basically considered to evaluations for the constitutions of the gel pack sheets, but the evaluation that the Example was better than the Comparative Example is considered to be derived from the facts that the body-side surface layer is good in ingredient release property and the environment-side surface layer has an appropriate closing effect (ODT effect) because the matrix of the body-side surface layer is sparser than the matrix of the environment-side surface layer. It is therefore considered that the synergy effect of the two surface layers may enhance the moisturizing effect.

The present invention has the following properties in consideration of the above test results and the other test results, and is very useful.

The combination of the flexible surface at the human body side with the environment-side surface having an appropriate flexibility and support property is very useful. Some actions should necessarily and repeatedly respond to deformation and bending in the surface of human body. When some deformation occurs in the surface of human body, the hydrous gel sheet should follow the deformation. When the deformation disappears in the human body, the hydrous gel sheet should also be immediately restored to the original state. If the hydrous gel sheet does not follow the deformation at the surface of human body, the user then feels a sense of incompatibility. If the hydrous gel sheet follows the deformation, but is not restored to its original state, the deformation is fixed to make blocking, wrinkle, and exfoliation. The problems such as skin incompatibility and deterioration of appearance will occur secondly. The product of the present invention is good in skin adhesiveness because of the flexibility of the body-side surface, good in the following property because of the appropriate flexibility of the environment-side surface, and also good in restoration because of the appropriate support property of the environment-side surface. Due to these reasons, the response to follow the deformation is extremely good and proper. On the contrary, the support body such as nonwoven fabric which has been used to the present time is not sufficiently good and proper in the response to follow the deformation.
·Existences of the differences in the adhesiveness of the two surfaces and in the flexibilities of the two surfaces can contribute to enhancement of handling properties. The existences of the differences make easiness on decision for handling method.
·The product of the present invention is good in stretching and deformation-absorbing properties. Stretching and deformation-absorbing properties along all directions are due to the gel sheet, particularly due to the hydrogel obtained from the reaction of the polyacrylic acid (or the salt thereof) with the multivalent metal ion.
·The matrix structure of the body-side surface layer is sparser, so it is good in ingredient release property and the matrix structure of the environment-side surface layer is denser, so it has ODT effect and contributes to enhance absorption of the ingredients into the skin.
·The appearance of the product is not basically conspicuous. When it is needed to be distinguished, a colorant may be used for the purpose.

### [Industrial Applicability]

The adhesive hydrous gel sheet used for adhering to a human body according to the present invention is good in appearance, aging stability and durability as well as stretching, deformation-absorbing, wear and handling properties, because it has not the support body such as nonwoven fabric but rather a support layer of the hydrogel at the environment-side surface.

## Claims

1. An adhesive hydrous gel sheet used for adhering to a human body comprising at least two laminated layers of a body-side surface layer and an environment-side surface layer, each of which is made of a hydrogel obtained from the reaction of polyacrylic acid or a salt thereof and a multivalent metal ion, wherein the polyacrylic acid or the salt thereof used for forming a gel matrix of the environment-side surface layer is at least 500,000 greater in weight-average molecular weight than the polyacrylic acid or the salt thereof used for forming a gel matrix of the human body-side surface layer.

2. The adhesive hydrous gel sheet as set forth in Claim 1, which has the property that when the adhesiveness of each of the two surface layers is measured with a slope type ball tack device (30 degree of slope angle) according to the test method for an adhesive tape or adhesive sheet described in JIS Z 0237, the difference of the ball number is 1 or more.

3. The adhesive hydrous gel sheet as set forth in one of Claims 1 and 2, in which the weight-average molecular weight of the polyacrylic acid or the salt thereof used for forming the gel matrix of at least one of the two surface layers is greater than 1,000,000.

4. The adhesive hydrous gel sheet as set forth in one of Claims 1 to 3, in which at least one water permeable core material is arranged in a layer of the two surface layers or/and between the two surface layers.

5. The adhesive hydrous gel sheet as set forth in one of Claims 1 to 4, in which at least one water impermeable core material is arranged in a layer of the two surface layers or/and between the two surface layers.

6. The adhesive hydrous gel sheet as set forth in one of Claims 1 to 5, in which at least one of the hydrogel layers includes oil gel dispersed into the hydrogel obtained by the reaction of the polyacrylic acid or the salt thereof with the multivalent metal ion.

7. The adhesive hydrous gel sheet as set forth in one of Claims 1 to 6, in which at least one of the hydrogel layers includes polysaccharide gel dispersed into the hydrogel obtained by the reaction of the polyacrylic acid or the salt thereof with the multivalent metal ion.
